# EUROPEAN PATENT SPECIFICATION

(11) **EP 0 132 403 B1**
(45) Date of publication and mention of the grant of the patent: **31.03.1993**
(21) Application number: 84304995.8
(22) Date of filing: 23.07.1984
(51) Int. Cl.: H02K 37/00

(54) **A surgically implantable device and stepping motor apparatus which may be used therein**
Gerät mit Schrittmotor zur Verwendung bei chirurgisch-implantierbaren Vorrichtungen
Dispositif chirurgical implantable et appareil moteur pas-à-pas utilisé dans celui-ci

(30) Priority: 21.07.1983 US 516137; 08.12.1983 US 559864; 08.12.1983 US 559865
(43) Date of publication of application: 30.01.1985
(60) Divisional application: 90202828.1
(73) Proprietor: Hakim, Salomon, Bogota (CO); Hakim, Carlos A., Fort Lauderdale Florida 33308 (US)
(72) Inventor: Hakim, Salomon, Bogota (CO); Hakim, Carlos A., Fort Lauderdale Florida 33308 (US)
(74) Representative: Deans, Michael John Percy

(56) References cited:
- EP-A- 0 060 369
- FR-A- 2 354 103
- US-A- 4 387 715

## Description

This invention relates to apparatus including stepping motors. Such apparatus may include shunt valves for venting cerebrospinal fluid ("CSF") in the treatment of hydrocephalus and similar conditions of impaired circulation and absorption of body fluids.

Cerebrospinal fluid shunt valves have been in use for over twenty years. Broadly speaking, they function by venting excess cerebrospinal fluid from the brain into the venous system or other receptive cavities (e.g., peritoneal, pleural). Many such valves, including the earliest designs, operate by controlling the amount of fluid flow. The neurosurgeon makes an estimate of the amount of flow required to relieve the hydrocephalus and selects a valve of that flow capacity. The selection is made difficult by the wide variation in normal flow rates.

About twenty years ago, one of the present applicants, namely Salomon Hakim, developed an altogether different valve, one that controlled intraventricular pressure rather than flow. That valve, which is today known as the Cordis-Hakim shunt valve, and which is described in U.S. Patent No. 3,288,142 has been enormously successful and remains, even today, one of the most popular shunt valves in use. It has a spherical sapphire ball biased against a conical valve seat by a stainless steel spring. The pressure of cerebrospinal fluid pushes against the sapphire ball and spring in a direction tending to raise the ball from the seat.

When the pressure difference across the valve (e.g., the pressure difference between the cerebral ventricle and the drainage site) exceeds a so-called popping pressure, the ball rises from the seat to vent cerebrospinal fluid. As the flow rate through the valve increases, the ball moves further away from the seat to provide a larger valve orifice, one that is always large enough that the pressure drop across the orifice never rises much above the popping pressure. Accordingly, the differential pressure across the valve remains nearly constant for any flow rate encountered within the cerebrospinal fluid system.

As successful as the Cordis-Hakim valve has been, it has one important limitation. It can only provide a fixed popping pressure. In treating hydrocephalus, it is often desirable to vary the popping pressure in accordance with ventricle size and treatment objective. For example, initial treatment may require a lower than normal pressure to initiate shrinkage of the ventricles, but as the ventricles decrease in size, the popping pressure should be increased gradually so that when the ventricles return to normal size the intraventricular pressure is at its normal value and the intracranial force systems are in balance (i.e., the popping pressure is set at a level that will stabilize the ventricles at a desired size). Generally speaking, the popping pressure should be varied inversely with the ventricle size. It is undesirable to leave a low pressure valve in a patient after the ventricles are again normal size, because the ventricles can further collapse, leading to a condition known as "slit" ventricles. A fuller discussion of these matters can be found in Hakim et al., "A Critical Analysis of Valve Shunts Used in the Treatment of Hydrocephalus", Developmental Medicine and Child Neurology, Vol. 15, No. 2, April 1973, pp. 230-255.

A further reason for providing adjustablility in popping pressure is to correct for the wide variation in nominal popping pressure typical in manufactured valves. With an adjustable valve, the popping pressure can be more accurately set at the factory, and can be checked, and corrected if necessary, in the operating room prior to implantation. Moreover it is unnecessary to manufacture and stock valves with differing nominal pressures, as one valve can typically provide all desired pressures according to the needs at any given moment of the treatment.

Efforts have been made at developing an adjustable valve. An example is a valve disclosed in our earlier-filed copending United States application Serial No. 493,748, in which an adjustment screw is turned either by a screw driver applied through the skin to the valve or by rotation of a magnet along an axis aligned with the axis of the screw.

Implantable magnetically-driven devices have been proposed. Levy et al. U.S. Patent No. 4,360,007 discloses an implantable actuator with a ratchet wheel, pawl, and permanent magnet; application of an external magnetic field rotates the implanted magnet and pawl to advance the ratchet wheel.

A magnetically operable hydrocephalus valve is disclosed by Sophysa in EP-A-0060369. A permanent bar magnet is rotatable from one position to another by the application of an external magnetic field. As the permanent bar magnet rotates further from an initial position the bias against a poppet valve provided by a flat strip attached to one end of the bar magnet is reduced. Detents are provided in the inner wall of the valve to cooperate with a projection on the other end of the bar magnet to mark successive angular positions.

Stepping motors have been known per se for many years. The simplest stepping motor consists of a permanent magnet rotor surrounded by a stator made up of four electromagnets. By selectively energizing the electromagnets, it is possible to turn the rotor in angular "steps" of 90°. Often the rotor has a plurality of permanent magnets, so as to reduce the angular step size. Some stepping motors replace the permanent magnets on the rotor with regions of different magnetic reluctance; in these, known as variable reluctance motors, the rotor turns to the position of minimum reluctance. Still others, known as hybrids, combine reluctance differences with permanent magnets.

Stepping motors have been used in some medical applications. For example, they have been used in medical infusion pumps for delivering precise volumetric dosages of drugs at prescribed time intervals. In all these applications the stepping motor was located outside the patient's body, either in a portable device carried by the patient or in a bedside unit.

Other types of medical devices (e.g., pacemakers) have been implanted in the body. These have typically relied for a power source on batteries implanted along with the device.

Heretofore stepping motors have required a power source, which means that for an implanted medical device there would have to be wiring extending through the skin or an implanted battery requiring periodic surgical intervention when the battery becomes exhausted.

The apparatus including a stepping motor provided by the present invention has arisen from the applicants' attempts to provide a better expedient to adjustability, for example of hydrocephalus valves or other implanted devices, than the prior magnetic arrangements but which did not require wiring through the skin or implanted batteries.

In accordance with the present invention we provide apparatus including a stepping motor comprising a rotor and one or more stator elements, the rotor being capable of movement relative to the stator element(s), characterised in that said stepping motor is isolated physically from electrical power sources and adapted to be powered by the influence of a magnetic field applied from outside the apparatus; and in that said stator element(s) is (are) composed of magnetically soft and permeable material, said material being so shaped and said material and said rotor being so positioned relative to each other that, when magnetized under the influence of said external field, the stator element(s) strengthen(s) and orient(s) the magnetic field in its (their) vicinity (vicinities) so as to cause incremental movement of said rotor.

The stepping motor may be installed in a surgically-implanted device and operated by application of a magnetic field applied from outside the body. The stepping motor may be operated while totally isolated physically from any source of electrical power (i.e.,without batteries and without wire connections to an external power source). The usual electromagnets used for the stator are replaced by pieces of magnetically soft and permeable material (e.g., pure iron or special alloys such as vacoperm). The externally applied magnetic field is used to magnetize the stator elements so that the localized magnetic field in the vicinity of the stator elements causes rotation of the rotor. The external magnetic field need not be applied with great precision, as the stator elements strengthen and orient the effect of the magnetic field in their vicinities.

In preferred embodiments, the rotor has a plurality of permanently magnetic poles; there are a plurality of stator elements spaced around the rotor or one stator element with a plurality of lobes so spaced; the external magnetic field is applied using apparatus having a plurality of electromagnets, which are equal in number to the stator elements (or lobes of one element) and positioned similarly; and the stator elements are composed either of soft and permeable material or of such material wrapped by an electrical coil (in which is induced an electrical current, which in turn helps magnetize the soft, permeable stator material). It should be understood that apparatus in accordance with the invention and including the stepping motor is not necessarily restricted to being surgically implantable.

Embodiments of apparatus in accordance with the invention have the advantage of allowing a mechanical movement (e.g., rotary or linear) to be induced within an implanted device (e.g., to alter the pressure setting of a valve, to activate a switch, or to vary the parameter of an electrical circuit) without any physical connection (e.g., electrical wires) with the device. It makes possible implanting a device with an internal element whose position can be accurately adjusted without the need for any wires, tubes, or other physical elements penetrating through the skin, or the use of implanted batteries. Possible applications include cerebrospinal fluid shunt valves (wherein the working pressure may be noninvasively adjusted very precisely) and implantable pumps for precise volumetric delivery of drugs.

In a preferred embodiment of our apparatus consisting of a surgically-implantable shunt valve for venting cerebrospinal fluid in the treatment of hydrocephalus or for shunting other body fluids, the apparatus further comprises: a housing constructed of a surgically-implantable material, an inlet and outlet chamber within said housing, said housing including inlet and outlet ports communicating with said inlet and outlet chambers respectively for connecting said inlet and outlet chambers to external catheters or other fluid conduits, an aperture communicating between said chambers, said aperture having a circular periphery forming a circular valve seat, a spherical ball of diameter larger than said circular valve seat, and spring means for biasing said ball against the circular valve seat, being adapted to keep said aperture closed until fluid pressure in said inlet chamber exceeds a preselected popping pressure and adapted to open said aperture when said popping pressure is exceeded so as to vent fluid through said aperture into said outlet chamber; and in that said stepping motor forms part of an incremental magnetic adjustment means for increasing or decreasing the amount of said bias in finite increments in response to pulses of an applied magnetic field so as thereby to increase or decrease said popping pressure in finite increments.

Such a valve has the advantages of immunity to the magnetic field generated by NMR (nuclear magnetic resonance) diagnostic devices; such devices generate a steady magnet field, which cannot cause more than about one incremental change of popping pressure.

The invention is hereinafter more particularly described by way of example only with reference to the accompanying drawings, in which:-
Fig. 1 is a perspective, somewhat diagrammatic, view of the preferred embodiment of our apparatus;
Fig. 2 is a cross-sectional view taken at 2-2 of Fig. 1 , showing the internal construction of said embodiment;
Fig. 3 is a cross-sectional view taken at 3-3 in Fig. 2;
Fig. 4 is an elevation view taken at 4-4 in Fig. 2;
Fig. 5 is a plan view at 5-5 in Fig. 2, showing the cam of said embodiment;
Fig. 6 is a cross-sectional view of said cam taken at 6-6 in Fig. 5;
Fig. 7 is a diagrammatic view of the steps of said cam;
Fig. 8 is a plan view of the internal support plate of said embodiment;
Fig. 8A is a cross-sectional view taken at 8A-8A in Fig. 8;
Fig. 9 is a plan view of the permanent-magnet disk of said embodiment, showing the ten pairs of poles on said disk;
Fig. 10 is a cross-sectional view of said disk taken at 10-10 in Fig. 9;
Fig. 11 is a diagrammatic view of said embodiment in which the positions of the external adjusting electromagnets are shown (much smaller than actual size);
Fig. 12 is a diagrammatic view of said embodiment implanted beneath the scalp and covered by an external adjustment element;
Fig. 13 is a diagrammatic view similar to Fig. 11 except that the rotor and cam have been removed to show the magnetic polarization of the four stator elements;
Figs. 14 and 15 show the magnetic polarization of an alternative embodiment, where a one-piece stator element is used;
Fig. 16 is a partial plan view, somewhat diagrammatic, of an alternative embodiment wherein the stator elements each include an electrical coil.

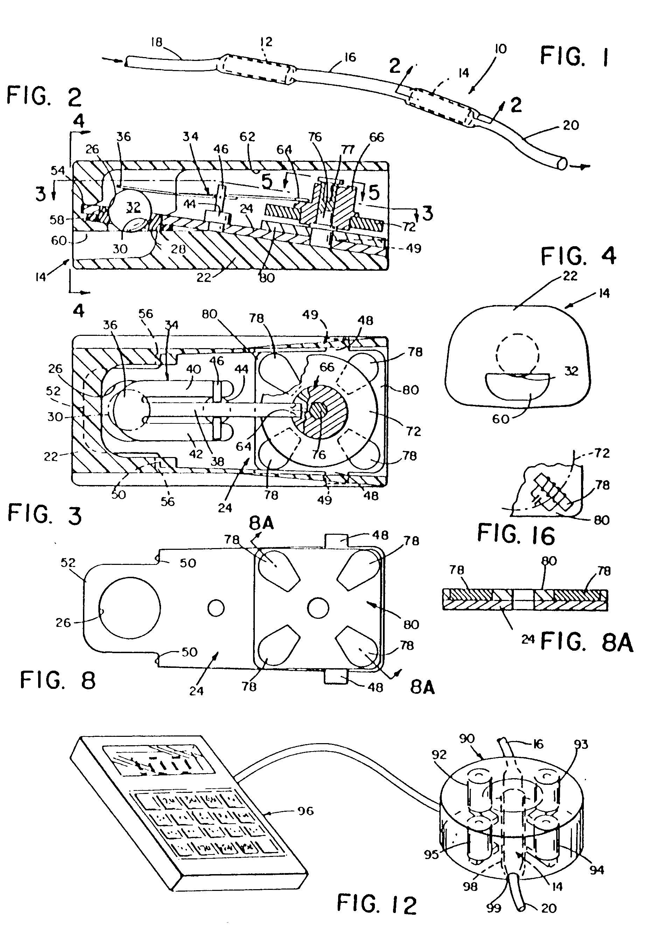

There is shown in Fig. 1 a shunt valve assembly 10 with two shunt valves 12, 14 separated by a pumping chamber 16. Cerebroventricular catheter 18 is connected to the inlet of the valve assembly, and drainage catheter 20, to the outlet. This assembly can be surgically implanted following well-known procedures.

A cross section through the downstream shunt valve 14 is shown in Fig. 2. The upstream valve 12 is preferably the same except that the adjustment mechanism is absent. (The tubular plastic covering shown tightly fitted around the valves in Fig. 17 is not shown in the remaining figures.) Valve body 22 (injection molded from a surgically-implantable material such as polyethersulfone) has within its interior an inclined plate 24 made from a nonmagnetic material, such as titanium or stainless steel. The plate 24 has circular aperture 26 in which is press fit a sapphire ring 28, with frustoconical surface 30 forming a valve seat for spherical ball 32 (highly-polished ruby).

Biasing the ball against the valve seat is spring 34 (single piece of stainless steel or another suitable material), shown in plan view in Fig. 3 . The spring provides a low K factor to produce little change in working pressure with changes in flow (i.e., a flat flow-pressure curve). The spring has base 36 overlying ball 32, central arm 38 extending from the base to an adjustment mechanism,, and two flanking arms 40, 42 extending from the base to a yoke 44. The yoke is press fit into a hole in plate 24 and tabs 46 extend over the tops of the flanking arms. The yoke is relieved in the center to provide room for the central arm to pass through. Notches (not shown) cut in the ends of flanking arms 40, 42 receive portions of the yoke, and secure the spring against longitudinal movement. The spring is secured against sideward movement by contact of the flanking arms with the vertical outside surfaces of the yoke.

Plate 24 is held tightly in place within valve body 22. The tight fit is achieved by sliding the plate into the valve body (in a direction from right to left in Fig. 2 . Grooves 54, 56 at the upstream end of the valve body receive portions 50, 52 (Fig. 8 of the plate, and grooves 49 at the downstream end receive tabs 48 on the plate. The grooves extend generally horizontally rather than in the inclined direction followed by the plate, and thus the tabs 48 and portions 50, 52 tend to become tightly wedged into the grooves.

Grooves 54, 56 at the ball end of the valve body also serve to press plate 24 downwardly so as to squeeze it tightly against O-ring 58 (silicone rubber), which provides an internal seal to ensure that all flow through the valve is through the orifice formed between the ball 32 and the valve seat 30. Flow through the valve is from inlet cavity 60, past ball 32, and into outlet cavity 62.

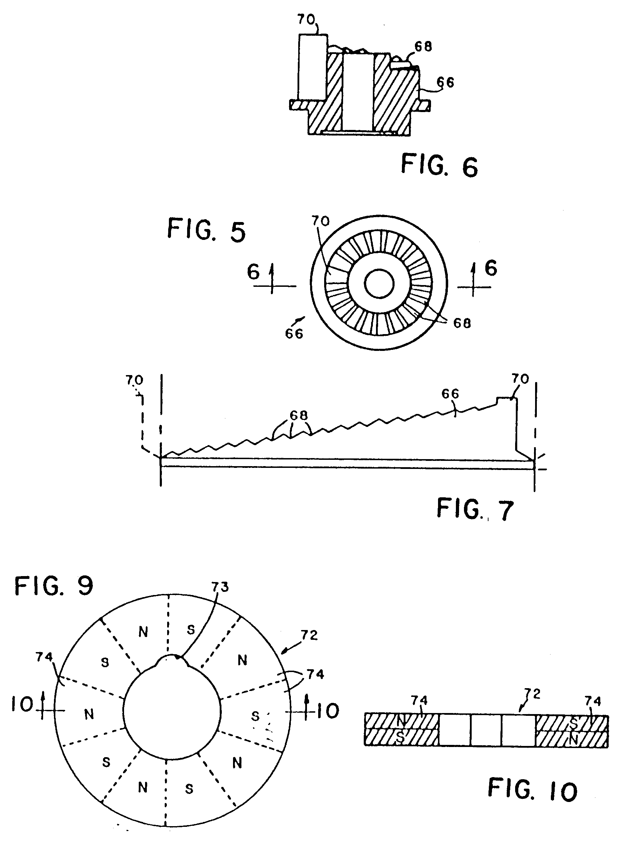

The preload of spring 34 against ball 32 is adjusted by using cam 66 (Delrin) to vary the vertical position (through a 0.75 mm range) of free end 64 of central arm 38. The spring preload establishes the pressure of the valve. The cam (best shown in Figs. 5-7) has a circular staircase of eighteen steps, each being grooved so as to have a V-shape cross section. Free end 64 of arm 38 has a similar V-shape chosen to mate with the V-shape of steps 68. At each end of the staircase a barrier is provided by element 70. This confines rotation of the cam to slightly less than one revolution. The V-shape of steps 68 act as detents to keep the cam in precisely one of eighteen possible angular positions. That means that the vertical position of free end 64 of arm 38 is always at precisely one of eighteen different values and, in turn, that the working pressure of the valve is always at one of eighteen possible levels.

Cam 66 is press fit into the central hole in rotor 72 (4 mm diameter), with a protrusion on the cam fitting into recess 73 in the rotor to assure accurate angular positioning. The cam-rotor unit rotates loosely on shaft 76, the base of which is press fit into plate 24. The unit is retained by retaining element 77 secured to the top of the shaft. The rotor is preferably made of platinum cobalt or samarium cobalt (which may be plated with platinum to improve corrosion resistance). The rotor has ten permanently magnetic poles 74 of alternate polarity (Figs. 9-10). At any one angular position, the pole exposed on the top surface of the disk is opposite that of the one exposed on the bottom surface.

Below rotor 72 there are fixed in place four stator elements 78 each made of a material that is magnetically soft and permeable, and that is resistant to corrosion in the presence of cerebrospinal fluid, which contains chlorides. Preferred materials include magnetic stainless steel alloys and alloys of nickel, iron, and molybdenum or cobalt. As shown in Fig. 8, the stator elements are embedded in a plastic member 80, which is fixed to plate 24 by means of shaft 76. The stator elements are shaped so that the portion of each lying beneath the rotor matches the size of permanent magnets 74. The portions of the stator elements lying radially beyond the rotor are sized to match the area beneath the rotor so that the boundary between poles, when the stator is magnetized, is at the perimeter of the rotor.

In operation the shunt valve assembly of Fig. 1 is surgically implanted in a patient following well-known procedures. Before implantation the pressure of adjustable valve 14 can be set to the desired level according to the circumstances of the case. For instance, it can be set approximately equal to the patient's pre-operative ventricular CSF pressure so that no immediate pressure change occurs as a result of the operation. After the patient has recovered from the trauma of the operation, the pressure is adjusted downwardly to the desired level. In the case of normal-pressure hydrocephalus, the pressure is lowered to a level sufficient to initiate shrinkage of the cerebral ventricle. Further adjustments in pressure can be made at subsequent times, as necessary. In the typical treatment of normal-pressure hydrocephalus, the pressure would be adjusted upwardly after sufficient shrinkage of the ventricle has occurred in order to stabilize ventricle size.

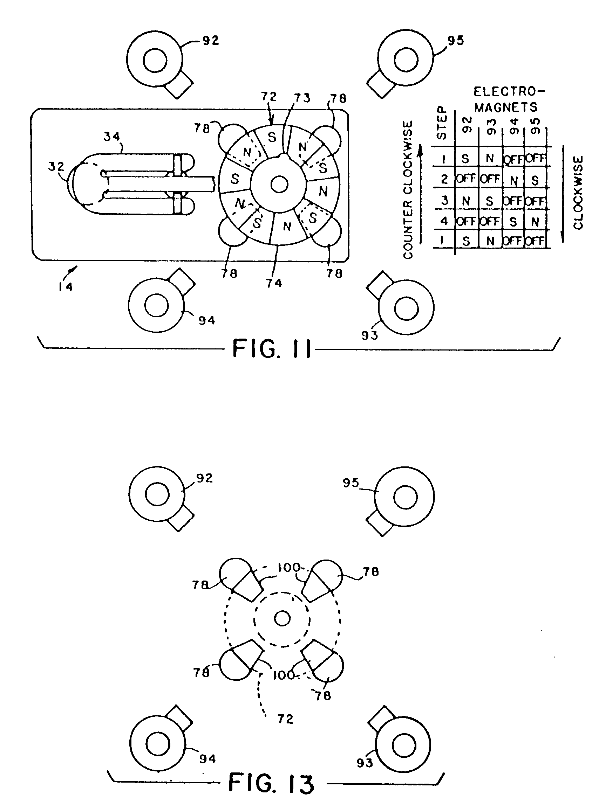

In children at the beginning of the treatment, the pressure should be lowered to a level inversely proportional to the ventricle size to reduce stress on the brain parenchyma (see Fig. 13 of Hakim et al., "The Physics of the Cranial Cavity", Surg. Neurol., Vol. 5, March 1976), and as the ventricle decreases in size the pressure of the valve should be increased, so when the ventricle attains normal size the intraventricular pressure is again normal, thereby avoiding development in the patient of a slit-ventricle condition. Also in cases of normal-pressure hydrocephalus, sometimes in spite of a low-pressure valve the patient does not improve and the ventricle size remains unchanged, making the surgeon think he is dealing with a case of brain atrophy. But by further changing the valve pressure to a lower one, the ventricle decreases in size and the patient immediately starts to improve. In elderly persons and in long standing cases of normal-pressure hydrocephalus, it has been found that the intraventricular pressure needs to be lowered more than in young people and in hydrocephalus of short duration.

Another advantage arises in the procedure for determining when an implanted shunt valve can safely be removed from a patient, i.e., determining whether the patient is still dependent on the valve for drainage of excess cerebrospinal fluid. The conventional technique for making that determination has been to temporarily pinch closed the tube downstream of the valve and observe the patient for symptoms (e.g., slight headache) indicative of valve dependency. In the absence of symptoms the valve can be removed. With the invention it is unnecessary to stop flow entirely. A safer procedure can be followed. Valve pressure is raised, slightly at first, more so later for confirmation, using the adjustment mechanism.

Valve pressure adjustments are made by applying a pulsed magnetic field to the vicinity of the shunt valve as shown diagrammatically in Figs. 11-13. A valve adjustment element 90 is applied over the adjustable valve 14 in the orientation shown. The adjustment element contains four electromagnets 92, 93, 94, 95, which are separately controlled by an external control device, shown diagrammatically at 96. Adjustment element 90 has a marking (such as an arrow pointing in the direction of CSF flow) on its exterior to assure that it is applied to the valve in the correct orientation, and it has a groove 98 in its bottom surface sized to fit over the protrusion in the scalp, at the site of the implanted valve. The groove is narrowed at one end 99 to enable correct longitudinal alignment relative to the adjustable valve 14.

Control device 96 has input keys, which the operator uses to select one of 18 possible desired pressure (from 20 to 190 mm H₂O) and a pressure display.

Each of electromagnets 92, 93, 94, 95 can be energized to have either the north or south polarity facing the stator elements, or each can remain off altogether. Movement of rotor 72, in the desired direction and through the desired angle, is achieved by energizing the electromagnets in the sequence shown in the table in Fig. 11. For example, clockwise motion is achieved by first energizing electromagnets 92, 93 to south and north polarities, respectively, and leaving electromagnets 94, 95 off. In the next step electromagnets 92, 93 are left off, and electromagnets 94, 95 are energized to north and south polarities. respectively. The sequence repeats itself after the fourth step. Rotor 72 is shown in Fig. 11 in the position reached after the first step (the polarities of the rotor magnets are those on the bottom surface). If the magnetic field provided by the electromagnets is described by a vector pointing from the south to the north pole of the energized magnets, then it can be seen that the sequence prescribed for causing rotor 72 to rotate clockwise (down the table in Fig. 11) amounts to rotating the field vector in the counterclockwise direction (opposite that of the rotor), in 90° steps.

Electromagnets 92, 93, 94, 95 are positioned 90° apart and spaced equal radial distances from a central axis. When adjustment device 90 is installed properly over valve 14, the central axis of the electromagnets is coincident with the axis of rotation of rotor 72, and each electromagnet is aligned at the same angular position as one stator element 78 . It is not, however, necessary that this alignment be exact. The invention is tolerant of alignment errors, which are unavoidable owing to the inability of the user to see rotor 72 or stator elements 78 and to the small size of those elements relative to the size of the external electromagnets.

The magnetic polarization induced in the stator elements 78 as the result of energizing the electromagnets is diagrammatically illustrated in Fig. 29. The two stator elements along the axis connecting the two energized electromagnets are polarized in the radial direction, so that the boundary between the poles lies roughly at the peripheral edge of disk rotor 72. The radially inner portions of these two stator elements, the portions lying beneath rotor 72, have the opposite polarity of the portions lying outside. By contrast, the stator elements along the other axis are polarized so that the boundary between poles lies along the radial direction. Both poles extend beneath the rotor 72. This pattern of polarization will result even if there is substantial error in the orientation of the electromagnets.

Movement of rotor 72 is influenced predominantly by the stator regions 100 (shown in dashed lines in Fig. 13) lying beneath the rotor, as it is those portions that are closest to the permanent magnets 74 of the rotor. Accordingly, the part of the stator elements with uniform polarity dominate over those with split polarity. This phenomenon could be emphasized by making the stator elements of a magnetically anisotropic material so that the magnetization induced by the external electromagnets is strongest along the radial axis of the corresponding stator elements.

The number of magnetic poles 74 is selected so that when one pair of radially opposite stator elements 78 is aligned with one pair of magnetic poles 74 (as are the upper left and lower right stator elements in Fig. 11) the other two stator elements (the upper right and lower left in Fig. 11) are each staggered halfway between two of the poles 74. In operation, control device 96 energizes the electromagnets closest to the pair of stators staggered between two magnets, thereby causing the rotor to move through an angle corresponding to one half the width of a magnetic pole 74.

In the preferred embodiment there are ten magnetic poles on each side of the disk, and thus twenty angular increments in one full revolution (i.e., each step is one twentieth of 360°, or 18°). Only eighteen of these increments are used, corresponding to the eighteen detented steps along the staircase surface of cam 68 (the other two increments are occupied by the detent wall 70 of the cam).

After a pressure is prescribed on control device 96, an enter key is pressed. That initiates a sequence of eighteen steps in the direction of lower pressure settings, counterclockwise rotation of rotor 72. This assures that the cam is returned to a position wherein spring arm 64 is at the lowest step on the cam staircase. If fewer than eighteen steps are actually needed to bring the cam to this position (as will most often be the case), the detent wall provided by element 70 of the cam prevents further rotation. After the eighteen-step resetting sequence is complete, the rotor is moved clockwise by the number of steps corresponding to the prescribed pressure.

Various variations can be made.

A magnetically anisotropic material could be used for the stator elements, with the strongest axis of magnetization oriented along the radial direction. Such anisotropy could also be achieved mechanically by splitting each stator element along the radial direction into two or more segments.

A variable reluctance or hybrid rotor could replace permanent-magnet rotor 72.

Linear movements within an implanted device could be achieved by providing a linearly-moving element as the rotor and by placing stator elements along the path of the linearly-moving rotor.

A rotor with fewer poles could replace the ten-pole rotor of the preferred embodiment, particularly where fine angular precision is not required (e.g., in a pump a simple two-pole rotor might suffice). Strong permanent magnets could be used to apply the external field (e.g., in the two-pole rotor of the pump application just described).

Electrical wire could be wrapped around the implanted stator elements forming a coil so that an electrical current is induced therein by the externally-pulsed magnetic field; the electrical current would in turn magnetize the stator elements if the coil circuit is closed by a resister or a capacitor.

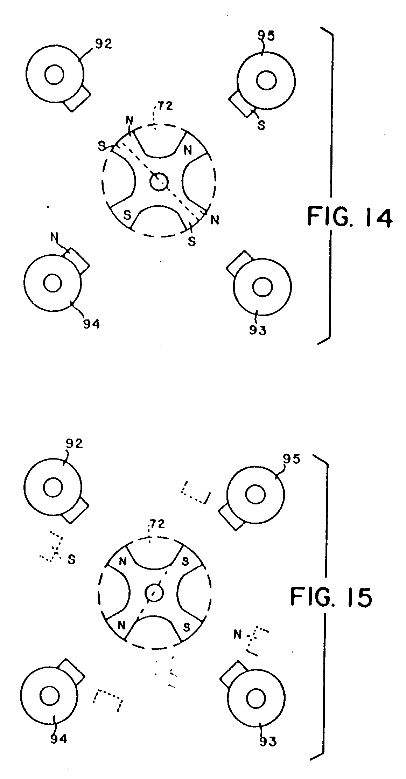

A single-piece stator element, e.g., with four lobes as shown in Fig. 14, could replace the stator elements of the preferred embodiment. An advantage of using a single-piece stator element is that the stator can lie entirely inside of the outer perimeter of the rotor, and thus provide for a more compact implanted unit. This is because, when magnetized, as shown in Fig. 30, the dominating lobes are all of one polarity, rather than split radially into two polarity regions as in the preferred embodiment. Thus the entire lobe, not just the inner half, can be influential in moving the rotor. The disadvantages of the single-piece stator is its lower tolerance to errors in alignment of the external magnetic field. The reduced tolerance for misalignment can be understood by reference to Fig. 15, in which the external field has been rotated sufficiently to cause the lobes that were split into two poles to now be entirely within the region of one pole. As a result all four lobes have nearly equal influence on the rotor, and it is not possible to move the rotor.

A presently even more preferred embodiment is one having a rotor 72 with six poles rather than ten (as shown in the figures). Such a rotor provides twelve steps of 30° for one full revolution. Eleven of these can be used to provide eleven different pressure settings (each differing by 15 mm H₂O) from 30 to 180 mm H₂O. An advantage of six poles is that with the four-stator-element configuration (Fig. 11) greater torque is available using six rather than ten poles. This results because all four stator elements, when magnetized by the external field, generates a torque in the same direction. In the ten-pole embodiment (Fig. 11), this is not the case. The torque generated by the stator elements with split polarity (upper right and lower left in Fig. 11) is opposite, though weaker than, that generated by the stator elements with uniform polarity. It is instructive to note, however, that this difference between the six and ten-pole embodiments is just the opposite if a one-piece, four-lobed stator is used. In that case, the split polarity stator elements generate opposing torque with the six-pole rotor and not with the ten-pole one. Also, if a ten-pole rotor is used with separate stator elements, torque can be increased by using anisotropic material for the stator elements, as that will increase the dominance of the uniform polarity stator elements over the split polarity ones.

## Claims

1. Apparatus including a stepping motor comprising a rotor and one or more stator elements, the rotor being capable of movement relative to the stator element(s), characterised in that said stepping motor is isolated physically from electrical power sources and adapted to be powered by the influence of a magnetic field applied from outside the apparatus; and in that said stator element(s) is (are) composed of magnetically soft and permeable material, said material being so shaped and said material and said rotor being so positioned relative to each other that, when magnetized under the influence of said external field, the said stator element(s) strengthen(s) and orient(s) the magnetic field in its (their) vicinity (vicinities) so as to cause incremental movement of said rotor.

2. Apparatus according to Claim 1, further characterised in that said apparatus is adapted to be surgically implanted, and said stepping motor is adapted when so implanted to be powered by the influence of a magnetic field applied from outside the body.

3. Apparatus according to Claim 1 or Claim 2, further characterised in that said rotor has permanently magnetic regions.

4. Apparatus according to any preceding claim, combined with external apparatus for generating said externally applied magnetic field, further characterised in that said external apparatus comprises a plurality of electromagnets and control means for selectively energizing said magnets in a sequence that causes said stator element(s) to be magnetized in successive angular steps causing the rotor to rotate.

5. Apparatus according to Claim 4, further characterised in that there are a plurality of stator elements angularly spaced about the axis of said rotor, and in that said electromagnets have the same angular spacing around a central axis as said stator elements have around the rotor axis so that said external apparatus can be positioned with said axes coincident and with said electromagnets at the same angular orientations as said stator elements.

6. Apparatus according to any preceding claim, further characterised in that there are a plurality of said stator elements spaced angularly around the perimeter of said rotor.

7. Apparatus according to any of Claims 1 to 5, further characterised in that said stator elements are provided as lobes extending from a common central portion, the lobes being spaced angularly around the perimeter of said rotor.

8. Apparatus according to any of Claims 1 to 5, further characterised in that there are either a plurality of said stator elements or angularly spaced stator element lobes joined in a common element, in that said stator elements or lobes are positioned so that particular regions thereof have greatest effect on movement of said rotor, and in that wherein said stator elements are positioned so that by variation in orientation in said externally applied magnetic field selected ones of said regions can be made either all one polarity or all the other polarity or split between both polarities, whereby the regions of one polarity can dominate over the regions of split polarity in controlling movements of said rotor.

9. Apparatus according to Claim 8, further characterised in that there are a plurality of said stator elements and each is positioned so that the radially inner half thereof is closest to the magnetically active regions of said rotor and thereby forms said region of greatest influence.

10. Apparatus according to Claim 8 or Claim 9, further characterised in that there are at least four said stator elements equally spaced around the perimeter of said rotor so that under the influence of an external magnetic field two radially opposing elements have said regions of all one polarity and the other two elements have said regions of split polarity.

11. Apparatus according to Claim 2 or any claim appendent thereto, further characterised in that said apparatus consists of a surgically-implantable shunt valve for venting cerebrospinal fluid in the treatment of hydrocephalus or for shunting other body fluids; in that said apparatus further comprises: a housing constructed of a surgically-implantable material, an inlet and outlet chamber within said housing, said housing including inlet and outlet ports communicating with said inlet and outlet chambers respectively for connecting said inlet and outlet chambers to external catheters or other fluid conduits, an aperture communicating between said chambers, said aperture having a circular periphery forming a circular valve seat, a spherical ball of diameter larger than said circular valve seat, and spring means for biasing said ball against the circular valve seat, being adapted to keep said aperture closed until fluid pressure in said inlet chamber exceeds a preselected popping pressure and adapted to open said aperture when said popping pressure is exceeded so as to vent fluid through said aperture into said outlet chamber; and in that said stepping motor forms part of an incremental magnetic adjustment means for increasing or decreasing the amount of said bias in finite increments in response to pulses of an applied magnetic field so as thereby to increase or decrease said popping pressure in finite increments.

## Patentansprüche

1. Vorrichtung mit einem Schrittmotor, letzterer umfassend einen Rotor und wenigstens ein Statorelement,
bei der der Rotor relativ zu dem (den) Statorelement(en) bewegbar ist,
dadurch gekennzeichnet,
daß der Schrittmotor körperlich (physikalisch) von elektrischen Spannungsquellen isoliert ist und so ausgestattet ist, daß er durch Einfluß (Induktion) eines Magnetfeldes, das außerhalb der Vorrichtung angeordnet ist, mit Energie versorgbar ist,
daß das (die) Statorelement(e) aus magnetisch weichem und permeablem Material bestehen, wobei das Material so geformt ist und der Rotor diesem gegenüber so plaziert ist, daß dann, wenn das (die) Statorelement(e) unter Einfluß des äußeren Feldes magnetisiert wird (werden),
dieses bzw. diese das in ihrer Nachbarschaft befindliche magnetische Feld verstärken und ausrichten und eine inkrementale Bewegung des Rotors bewirken.

2. Vorrichtung nach Anspruch 1, dadurch gekennzeichnet, daß die Vorrichtung chirurgisch implantierbar ist und daß der Schrittmotor im implantierten Zustand durch Einfluß eines Magnetfeldes, das von außerhalb des Körpers wirkt, mit Energie versorgbar ist.

3. Vorrichtung nach Anspruch 1 oder 2, dadurch gekennzeichnet, daß der Rotor permanentmagnetische Bereiche aufweist.

4. Vorrichtung nach einem der vorhergehenden Ansprüche, kombiniert mit einer externen Vorrichtung zur Erzeugung des von außen angelegten Feldes, dadurch gekennzeichnet, daß die externe Vorrichtung eine Vielzahl von Elektromagneten und Steuerungsvorrichtungen umfaßt, um die genannten Magnete selektiv in einer Sequenz zu induzieren, die die Statorelemente in sukzessiven Winkelschritten magnetisiert und damit den Rotor zum Drehen veranlaßt.

5. Vorrichtung nach Anspruch 4, dadurch gekennzeichnet, daß eine Vielzahl von Statorelementen vorhanden ist, die in Winkelabständen um die Rotorachse angeordnet sind, und daß die genannten Elektromagnete denselben Winkelabstand um eine Mittelachse haben wie die genannten Statorelemente um die Rotorachse, so daß die externe Vorrichtung in eine koinzidierende Position der Achsen gebracht werden kann, bei der die Elektromagnete dieselbe Winkelorientierung wie die Statorelemente haben.

6. Vorrichtung nach einem der vorhergehenden Ansprüche, dadurch gekennzeichnet, daß eine Vielzahl von Statorelementen vorhanden ist, die in Winkelabständen um den Rotor-Umfang angeordnet sind.

7. Vorrichtung nach einem der vorhergehenden Ansprüche 1 bis 5, dadurch gekennzeichnet, daß die Statorelemente in Nierenform (lobes) gestaltet sind, die sich von einer gemeinsamen Zentralposition erstrecken und bei der die Nierenformen in Winkelabständen um den Rotor-Umfang angeordnet sind.

8. Vorrichtung nach einem der vorhergehenden Ansprüche 1 bis 5, dadurch gekennzeichnet, daß entweder eine Vielzahl derartiger Statorelemente oder nierenförmiger, in Winkelabständen angeordneter Statorelemente, die zu einem gemeinsamen Element verbunden sind, vorhanden ist, und daß die Statorelemente oder Nierenformen derartig angeordnet sind, daß bestimmte Abschnitte davon den größten Effekt auf die Bewegung des Rotors ausüben,
und dadurch, daß die Statorelemente so angeordnet sind, daß bei Veränderung der Orientierung des genannten, von außen angelegten Magnetfeld bestimmte, ausgewählte Abschnitte entweder alle in einer ersten Polarität oder alle in der anderen Polarität ausgerichtet werden können oder daß zwischen den Polaritäten aufgeteilt werden kann, wobei Abschnitte der einen Polarität über die Abschnitte der anderen Polarität bei der Steuerung des Rotors dominieren können.

9. Vorrichtung nach Anspruch 8, dadurch gekennzeichnet, daß eine Vielzahl von Statorelementen vorhanden ist und daß jedes Statorelement so angeordnet ist, daß seine in Radiusrichtung innere Hälfte dem magnetisch aktiven Abschnitt des Rotors am nächsten ist und dabei einen Bereich größter Induktion bildet.

10. Vorrichtung nach Anspruch 8 oder 9, dadurch gekennzeichnet,daß wenigstens vier derartiger Statorelemente vorhanden sind, die in gleichen Winkelabständen um den Umfang des Rotors angeordnet sind, so daß unter dem Einfluß des äußerlich angelegten magnetischen Feldes zwei radial sich gegenüberliegende Elemente die besagten Bereiche einzig die erste Polarität und die beiden anderen Elemente die Abschnitte aufgeteilter Polarität aufweisen.

11. Vorrichtung nach Anspruch 2 oder nach einem der zu Anspruch 2 nachgeordneten Ansprüche, dadurch gekennzeichnet,daß die Vorrichtung aus einem chirurgisch implantierbaren Nebenschluß-Ventil zur Ableitung (Ventilierung) cerebrospinalen Fluids bei der Behandlung des Hydrocephalus (Wasserkopf) oder zur zur Ableitung anderer Körperflüssigkeiten dient, wobei die Vorrichtung weiterhin umfaßt:
ein Gehäuse, das aus chirurgisch implantierbarem Material besteht,
eine Einlaß- und eine Auslaßkammer innerhalb des Gehäuses, wobei das Gehäuse Einlaß- und Auslaßkanäle besitzt,
über die die Einlaß- bzw. Auslaßkammer mit externen Kathetern oder anderen FLüssigkeitsleitungen verbindbar ist,
eine Öffnung, die mit beiden Kammern verbunden ist und die eine ringförmige Peripherie aufweist, die einen Ventil-Ringsitz bildet,
eine Kugel, deren Durchmesser größer als der Ventil-Ringsitz ist,
und Federmittel, die die Kugel gegen den ringförmigen Ventil-Ringsitz drückt und die so eingestellt ist, daß die Öffnung geschlossen bleibt, bis der Flüssigkeitsdruck in der Einlaßkammer einen voreingestellten Öffnungsdruck überschreitet, und die die Öffnung öffnet,
wenn der Öffnungsdruck überschritten ist, so daß Flüssigkeit durch die Öffnung in die Auslaßkammer abführbar ist,
und dadurch, daß der Schrittmotor einen Teil einer inkrementalen magnetischen Einstellvorrichtung bildet, um den Wert des Federdrucks in finiten Schritten in Reaktion auf die Impulse eines angelegten magnetischen Feldes zu erhöhen oder zu erniedrigen, so daß der Öffnungsdruck in finiten Schritten erhöht oder erniedrigt wird.

## Revendications

1. Appareil comprenant un moteur pas à pas constitué par un rotor et un ou plusieurs éléments de stator, le rotor étant suceptible de subir un mouvement relatif par rapport à l'élément ou aux éléments du stator, caractérisé en ce que ledit moteur pas à pas est physiquement isolé des sources d'énergie électrique et prévu pour être alimenté sous l'effet d'un champ magnétique extérieur à l'appareil; et en ce que le ou lesdits élément(s) du stator est (sont) constitué(s) d'un matériau magnétiquement doux et perméable, le matériau ainsi formé ainsi que le rotor étant disposés l'un par rapport à l'autre, de façon que sous l'effet du champ extérieur, le ou lesdits élément(s) du stator renforcent et orientent le champ magnétique dans son (leur) voisinage de manière à créer un mouvement incrémentiel du rotor.

2. Appareil selon la revendication 1, caractérisé en outre en ce que ledit appareil est conçu pour une implantation chirurgicale et en ce que ledit moteur pas à pas est conçu pour être alimenté, après implantation, par l'effet d'un champ magnétique appliqué depuis l'extérieur du corps du malade.

3. Appareil selon la revendication 1 ou 2, caractérisé en outre en ce que ledit rotor comprend des zones magnétiques permanentes.

4. Appareil selon l'une quelconque des revendications précédentes, associé à un appareil extérieur pour générer le champ magnétique appliqué extérieurement, caractérisé en outre en ce que ledit appareil extérieur comprend plusieurs électro-aimants et des moyens de commande pour exciter sélectivement lesdits aimants suivant une séquence qui force le ou lesdits élément(s) du stator à être aimanté(s) par pas angulaires successifs entraînant la rotation du rotor.

5. Appareil selon la revendication 4, caractérisé en outre en ce qu'il comporte plusieurs éléments de stator espacés selon un certain angle autour de l'axe du rotor, et en ce que les électro-aimants ont autour d'un axe central le même écartement angulaire que les éléments de stator autour de l'axe du rotor de manière que l'appareil extérieur puisse coïncider avec lesdits axes et avec lesdits électro-aimants sous les mêmes orientations angulaires que lesdits éléments de stator.

6. Appareil selon l'une quelconque des revendications précédentes, caractérisé en outre en ce qu'il comprend plusieurs éléments de stator espacés selon un certain angle autour du périmètre du rotor.

7. Appareil selon l'une quelconque des revendications 1 à 5, caractérisé en outre en ce que les éléments du stator sont montés sous forme de lobes partant d'une position centrale commune, les lobes étant angulairement espacés autour du périmètre du rotor.

8. Appareil selon l'une quelconque des revendications 1 à 5, caractérisé en outre en ce qu'il comprend soit plusieurs éléments de stator, soit des éléments de stator en lobes espacés angulairement et réunis dans un élément commun, en ce que lesdits éléments ou lobes du stator sont disposés de façon que certaines de leurs zones aient un effet très important sur le mouvement du rotor, et en ce que les éléments de stator sont disposés de telle manière qu'en faisant varier leur orientation dans le champ magnétique extérieur, certaines zones sélectionnées puissent soit toutes avoir la première polarité, soit toutes avoir l'autre polarité, soit être partagées entre les deux polarités, de façon que les zones de la première polarité puissent dominer les zones ayant une polarité partagée pour commander les mouvements du rotor.

9. Appareil selon la revendication 8, caractérisé en outre en ce qu'il existe plusieurs éléments de stator et en ce que chacun d'eux est placé de manière que sa moitié située radialement vers l'intérieur soit très rapprochée des zones magnétiquement actives du rotor et constitue ainsi la zone d'influence prépondérante.

10. Appareil selon la revendication 8 ou 9, caractérisé en outre en ce qu'il existe au moins quatre éléments de stator également répartis autour du périmètre dudit rotor de façon que sous l'effet d'un champ magnétique extérieur, deux éléments radialement opposés correspondent aux zones ayant toutes une première polarité et les deux autres éléments correspondent aux zones dont la polarité est partagée.

11. Appareil selon la revendication 2 ou l'une quelconque des revendications dépendantes de celle-ci, caractérisé en outre en ce que ledit appareil est constitué par une valve en dérivation implantable par opération chirurgicale pour faire communiquer avec l'extérieur le liquide céphalo-rachidien au cours du traitement d'une hydrocéphalie ou pour shunter d'autres fluides corporels ; en ce que ledit appareil comprend en outre : un boîtier construit en matériau implantable par opération chirurgicale, une chambre d'entrée et une chambre de sortie à l'intérieur dudit boîtier, ledit boîtier comprenant des orifices d'entrée et de sortie en communication respective avec les chambres d'entrée et de sortie pour relier lesdites chambres à des cathéters extérieurs ou à d'autres conduits de fluide, une communication étant prévue entre lesdites chambres, cette ouverture étant de forme circulaire et constituant le siège circulaire d'une valve, une bille sphérique d'un diamètre supérieur à celui du siège circulaire de ladite valve, et un ressort pouvant solliciter ladite bille en l'appuyant contre le siège circulaire de la valve, étant conçu pour maintenir l'ouverture fermée jusqu'à ce que la pression du fluide contenu dans la chambre d'entrée dépasse la pression pré-sélectionnée de commande de la valve et ouvre celle-ci de manière à laisser passer le fluide par l'ouverture ménagée dans la chambre de sortie ; et en ce que ledit moteur pas à pas fait partie d'un moyen de réglage magnétique par incrément ayant pour objet d'augmenter ou de diminuer l'amplitude de ladite sollicitation par incréments finis en réponse aux impulsions d'un champ magnétique appliqué de façon à augmenter ou diminuer ainsi ladite pression de déclenchement par incrément fini.
